# EUROPEAN PATENT APPLICATION

(11) **EP 3 622 939 A2**
(43) Date of publication of application: **18.03.2020**
(21) Application number: 18799244.1
(22) Date of filing: 08.05.2018
(51) Int. Cl.: A61H 39/04, A61F 7/00

(54) **EAR THERAPY DEVICE**

(30) Priority: 08.05.2017 KR 20170057559
(71) Applicant: Seo, Jong-Soo, Seongnam-si, Gyeonggi-do 13166 (KR)
(72) Inventor: Seo, Jong-Soo, Seongnam-si, Gyeonggi-do 13166 (KR)
(74) Representative: advotec.
(86) International application number: PCT/KR2018/005277
(87) International publication number: WO 2018/208063

(57) **Abstract**

Disclosed is an ear therapy device, which performs massage to an external ear by using a massage module, which includes a housing having a facing portion facing the external ear and a plurality of accommodation holes divided therein by a barrier; a plurality of fluid filling bags dispersed and accommodated in the plurality of accommodation holes, respectively; and a plurality of stimulation pins disposed inside the housing such that at least one stimulation pin corresponds to each of the plurality of fluid filling bags.

## Description

### TECHNICAL FIELD

The present application claims priority to Korean Patent Application No. 10-2017-0057559 filed on May 8, 2017 in the Republic of Korea, the disclosures of which are incorporated herein by reference.

The present disclosure relates to an ear therapy device, and more particularly, to an ear therapy device for massaging an external ear among the body of a human.

### BACKGROUND ART

Generally, a meridian point is a specific portion on the body skin that requires medical diagnosis and treatment. In particular, it is known that auricular meridian points that correspond to the meridian points of the ear are connected to various organs of the body to interact therewith. Accordingly, in the oriental medicine, ear therapy that is so-called auricular acupressure is used as one of the disease treatment methods. The ear therapy is a treatment that determines the state of health and constitution of the body by observing various positive reactions exhibited on the ear and then stimulates appropriate auricular meridian points to facilitate blood circulation and enhance immunity and homeostasis of the body.

Recently, various techniques for massaging ears or stimulating auricular meridian points have been proposed according to the ear therapy.

However, as disclosed in Korean Unexamined Patent Publication No. 10-2006-0013338 and the like, in the existing technologies, a current is sent to a protrusion formed at a fixed position and having a fixed length to stimulate the ear of the user. Thus, it is impossible to implement an actual massage of stimulating the external ear of the user with an acupressure tool having a pin or stick shape. Also, it is impossible to adaptively operate according to the shape or structure of the external ear which is different for each user.

In addition, as disclosed in Korean Utility Model Registration No. 20-0473957, in the existing technology, the external ear of the user is stimulated through air tubes that inflated by the air pressure. Thus, when the stimulation is actually implemented, the air tubes have different expansion rates, and also various parts of each air tube also have different expansion rates, which makes it difficult to accurately stimulate a specific point. In addition, the elastic force of the air tube is reduced by repeated use, resulting in deformation of the air tube. That is, in the existing technologies, the accuracy and uniformity of the stimulating operations to a specific point or a specific part of the external ear are inferior. Moreover, the existing technologies do not provide a way for a user to reflect his/her health status or medical examination result to the massage scenario.

### DISCLOSURE

### Technical Problem

The present disclosure is directed to providing an ear therapy device, which may massage an external ear of a user in the same way as an actual massage using an acupressure tool, adaptively operate according to the shape and structure of the external ear, which is different for each user, and improve the accuracy and uniformity of the stimulation operation.

### Technical Solution

In one aspect of the present disclosure, there is provided an ear therapy device, which performs massage to an external ear of a user by using a massage module disposed at a position corresponding to the external ear, wherein the massage module includes: a housing having a facing portion facing the external ear and a plurality of accommodation holes divided therein by a barrier; a plurality of fluid filling bags dispersed and accommodated in the plurality of accommodation holes, respectively; and a plurality of stimulation pins disposed inside the housing such that at least one stimulation pin corresponds to each of the plurality of fluid filling bags, wherein a plurality of guide holes are formed in the facing portion of the housing in one-to-one relationship with plurality of stimulation pins, wherein each fluid filling bag has a bellows structure with a length increased or decreased in a predetermined direction according to a pressure of a fluid filled therein, and wherein each stimulation pin is pushed by a corresponding fluid filling bag and drawn out of the housing by a predetermined length when the length of the corresponding fluid filling bag is increased, and the each stimulation pin is guided by a corresponding guide hole to be drawn in a predetermined direction.

In an embodiment, the housing may have a plurality of fluid delivery passages configured to independently deliver a fluid supplied from the outside to the plurality of fluid filling bags, respectively.

In an embodiment, each fluid filling bag may include an opening connected to the fluid delivery passage of the housing; a bellows structure having a length increased or decreased in a predetermined direction according to the pressure of the fluid delivered through the opening; and a support provided to a terminal of the bellows structure to support a corresponding stimulation pin.

In an embodiment, each stimulation pin may include a head supported in contact with the corresponding fluid filling bag; and a rod having one end connected to the head and extended in one direction so that the other end thereof is located in the corresponding guide hole.

In an embodiment, at least one of the plurality of stimulation pins may further include an auricular meridian point stimulating cap detachably coupled to the other end of the rod.

In an embodiment, the plurality of stimulation pins may be grouped and disposed into a plurality of stimulation pin groups to respectively correspond to a plurality of unit areas formed by dividing an external ear area according to stimulation effects or positions, and the lengths of the plurality of fluid filling bags may be independently increased by the fluid supplied from the outside to draw the plurality of stimulation pins for each stimulation pin group.

In an embodiment, the facing portion of the housing may include a protrusion protruding toward the external ear so that a part of the plurality of guide holes is located therein, and among the plurality of stimulation pins, a stimulation pin drawn through the guide hole located in the protrusion may have a greater length than another stimulation pin drawn through a guide hole not in the protrusion.

In an embodiment, the massage module may further include an elastic body configured to insert the drawn stimulation pin into the housing by an elastic force thereof, when the length of the corresponding fluid filling bag pushing the stimulation pin drawn out of the housing is decreased.

In an embodiment, the housing may include a first housing in which the plurality of accommodation holes are formed to accommodate the plurality of fluid filling bags; and a second housing having the facing portion in which the plurality of guide holes are formed, the second housing being detachably coupled to the first housing to form an inner space in which the plurality of stimulation pins are accommodated.

In an embodiment, the first housing may include a plurality of sub housings stacked and coupled in order, and the plurality of sub housings may include a first sub housing in which at least one first accommodation hole is formed to accommodate the fluid filling bag; and a second sub housing in which a penetration hole connected to the first accommodation hole and at least one second accommodation hole accommodating the fluid filling bag are formed, the second sub housing being stacked on the first sub housing.

In an embodiment, the ear therapy device may further comprise a wearable structure coupled to the massage module and put on the head of the user so that the massage module is disposed at a position corresponding to the external ear of the user; and a heating unit configured to transfer heat to the external ear using a hot wire or a far-infrared lamp.

In an embodiment, the ear therapy device may further comprise an input unit configured to receive a user command to select an operation mode of the ear therapy device; a pressure generating unit configured to move a fluid by using a pump; a flow path opening and closing unit configured to open or close each fluid moving path connecting the pressure generating unit and the massage module by using a valve; a storing unit configured to store a fluid filling bag serving as an operation target according to each operation mode of the ear therapy device and operation information about operation contents of the corresponding fluid filling bag; and a control unit configured to control the flow path opening and closing unit according to the operation information corresponding to the operation mode selected through the input unit among the operation information of each operation mode stored in the storing unit, so that the fluid moving paths respectively connected to the plurality of fluid filling bags are selectively opened or closed.

In an embodiment, the pressure generating unit may include a pump configured to suck in a fluid through an input terminal and discharge the fluid through an output terminal; and a pressure control bag installed to a fluid moving path between the output terminal of the pump and the flow path opening and closing unit and expanded or shrunk to limit a pressure change rate, generated at the output terminal of the pump when the flow path opening and closing unit performs the opening or closing operation, to a predetermined threshold value or below.

In an embodiment, the storing unit may store an operation information table in which information about a fluid filling bag serving as an operation target according to each operation mode, stimulation intensity and stimulation time is stored.

In an embodiment, the ear therapy device may further comprise a pressure sensor configured to detect a pressure generated by the pressure generating unit, and the control unit may control the pressure generating unit according to the pressure value detected by the pressure sensor.

The embodiments according to the present disclosure may be implemented using a computer program that executes the above-described operations or methods through a computer system.

### Advantageous Effects

According to the present disclosure, since the stimulation pins accommodated in the massage module are drawn with the appropriate length and pressure by the fluid filling bag to stimulate the external ear of the user, the ear therapy device may massage the external ear of the user in the same way as the actual massage using an acupressure tool, and also the ear therapy device may adaptively operate according to the shape or structure of the external ear that is different for each user.

In addition, instead of simply expanding the fluid filling bags to push the stimulation pins, the stimulation pins are pushed in a predetermined direction using a bellows structure having a length increased or decreased in a predetermined direction according to the pressure of the fluid filled therein, and the guide holes of the housing guide the drawing direction of the stimulation pins pushed by the fluid filling bags. Thus, it is possible to improve the accuracy and uniformity of the stimulation operation using the stimulation pins.

In addition, the plurality of stimulation pins accommodated in the massage module are grouped into a plurality of groups to correspond to the plurality of unit areas formed by dividing the external ear area of the user according to the stimulation effects or positions, and the stimulation pins of each group are drawn according to an operation mode selected by the user to stimulate the external ear of the user. Thus, the user may reflect his/her health status or medical examination results to a massage scenario.

In addition, since the auricular meridian point stimulating module, which accommodates the stimulation pins and faces the external ear of the user, is configured to be detachably coupled with the operating module accommodating the fluid filling bags, the auricular meridian point stimulating module may be easily exchanged and customized for each user, and the ear therapy device may be hygienically used and managed even when being commonly used in public facilities such as medical institutions.

In addition, since the pressure control bag is installed to the output terminal of the pump that delivers the fluid to the fluid filling bag, it is possible to prevent the generation of noise of the pump caused by a rapid pressure change when opening and closing the fluid moving path.

In addition, since the heating unit of the ear therapy device transfers heat to the external ear of the user through a hot wire or a far-infrared lamp, it is possible to give a thermal massage effect through the ear therapy device.

Further, it would be obviously understood from the following description by those skilled in the art that the embodiments according to the present disclosure can also solve various technical objects not mentioned above.

### DESCRIPTION OF DRAWINGS

FIG. 1 is a perspective view showing an ear therapy device according to an embodiment of the present disclosure.
FIG. 2 is a front perspective view showing a massage module of the ear therapy device according to an embodiment of the present disclosure.
FIG. 3 is a rear front perspective view showing the massage module depicted in FIG. 2.
FIG. 4 is a vertical sectioned view, taken along the line A-A' of FIG. 2.
FIG. 5 is a front perspective view showing a massage module of an ear therapy device according to another embodiment of the present disclosure.
FIG. 6 is a rear front perspective view showing the massage module depicted in FIG. 5.
FIG. 7 is a front view showing the massage module depicted in FIG. 5.
FIG. 8 is an exploded perspective view showing the massage module depicted in FIG. 5.
FIG. 9 is a vertical sectioned view, taken along the line B-B' of FIG. 7.
FIGS. 10 and 11 are a front perspective view and a rear front perspective view respectively showing a first layer structure of a first housing.
FIGS. 12 and 13 are a front perspective view and a rear front perspective view respectively showing a second layer structure of the first housing.
FIG. 14 is a diagram showing a portion X of FIG. 9.
FIG. 15 is a diagram showing a drawing operation of the stimulation pin.
FIG. 16 is a diagram showing a modified example of the stimulation pin.
FIG. 17 is a block diagram showing the ear therapy device according to an embodiment of the present disclosure.
FIG. 18 is a diagram showing a fluid moving path of the ear therapy device according to an embodiment of the present disclosure.
FIG. 19 is a diagram showing a fluid moving path of an ear therapy device according to another embodiment of the present disclosure.
FIG. 20 is a diagram showing an example of stimulation target areas formed by dividing the external ear area of a human according to stimulation effects.
FIG. 21 is a diagram showing an example of an operation information table stored in a storing unit.

### BEST MODE

Hereinafter, embodiments of the present disclosure will be described in detail with reference to the accompanying drawings in order to clarify a solution to the technical problem of the present disclosure. However, in explaining the present disclosure, related known technologies will not be explained if the gist of the present disclosure becomes obscure due to the explanation. In addition, the following terms are defined in consideration of functions in the present disclosure, which may vary depending on the intention or custom of a designer, a manufacturer, or the like. Therefore, the definition should be based on the contents throughout this specification.

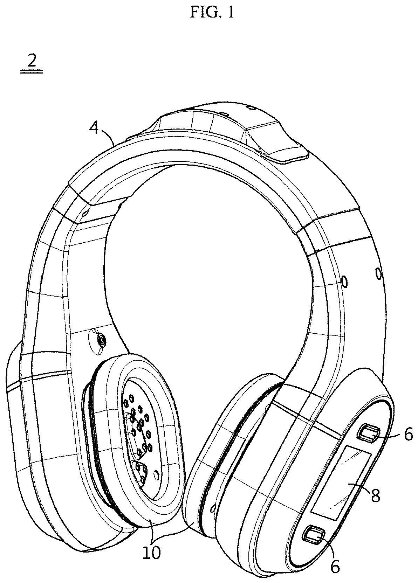

FIG. 1 is a perspective view showing an ear therapy device 2 according to an embodiment of the present disclosure.

As shown in FIG. 1, the ear therapy device 2 according to the present disclosure performs massage to an external ear of a user by using a massage module 10 disposed at a position corresponding to the external ear. In this case, the massage module 10 may be supported by various structures and disposed at the external ear of the user.

For example, the ear therapy device 2 may include a wearable structure 4. The wearable structure 4 is coupled with the massage module 10 and put on the head of the user so that the massage module 10 is disposed at a position corresponding to the external ear of the user. The wearable structure 4 may be configured in various forms. For example, as shown in FIG. 1, the wearable structure 4 may be configured in the form of a head band. Depending on embodiments, the wearable structure 4 may be configured in the form of a helmet. An input unit 6 used to turn on/off the power of the ear therapy device 2 or set an operation mode or the like of the ear therapy device 2 and a display unit 8 for visually displaying information of the ear therapy device 2 such as a current operation mode, stimulation intensity, stimulation time and a battery state may be installed at an outer surface of the wearable structure 4.

The massage module 10 accommodates a plurality of stimulation pins 16 inside the housing thereof, and may stimulate the external ear by selectively drawing the accommodated stimulation pins to contact the external ear at a predetermined pressure.

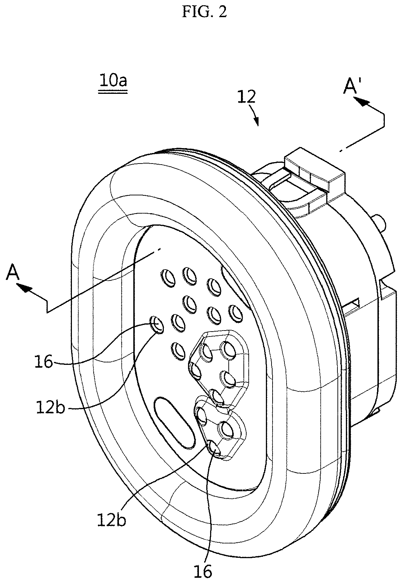

FIG. 2 is a front perspective view showing a massage module 10a of the ear therapy device according to an embodiment of the present disclosure.

As shown in FIG. 2, the massage module 10a includes a housing 12 and stimulation pins 16.

The housing 12 has a facing portion facing the external ear of the, and the facing portion has a guide hole 12b formed therein so that the stimulation pin 16 accommodated inside the housing 12 is drawn therethrough. The plurality of stimulation pins 16 may be accommodated in the housing 12 and disposed at predetermined positions. In this case, a plurality of guide holes may be formed in the facing portion of the housing 12 in a one-to-one relationship with the plurality of stimulation pins.

In an embodiment, the facing portion of the housing 12 may include a protrusion protruding toward the external ear of the user. The protrusion may be formed at a position corresponding to an area around the ear hole, which is located relatively deep in the entire area of the external ear. A part of the plurality of guide holes formed in the facing portion may be located in the protrusion. In this case, among the plurality of stimulation pins accommodated in the housing 12, a stimulation pin drawn out through the guide hole located in the protrusion may have a greater length than another stimulation pin drawn out through the guide hole not located in the protrusion. The reason why the stimulation pin disposed at the protrusion position is relatively longer than the stimulation pin disposed at another position as described above is to stimulate the area around the ear hole located at a relatively deep position with an appropriate pressure.

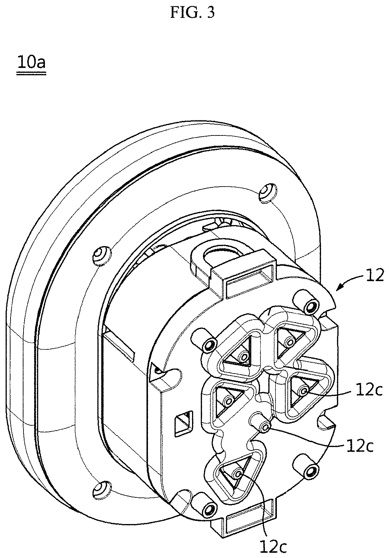

FIG. 3 is a rear front perspective view showing the massage module 10a depicted in FIG. 2.

As shown in FIG. 3, the housing 12 of massage module 10a has a fluid delivery passage 12c in a portion other than the facing portion that faces the external ear of the user. As will be described again below, the fluid delivery passage 12c is configured to deliver a fluid, such as air or oil, supplied from the outside to a fluid filling bag accommodated in the housing 12. A plurality of fluid filling bags may be accommodated in the housing 12, and in this case, the housing 12 may have a plurality of fluid delivery passages for independently delivering the fluid supplied from the outside to the plurality of fluid filling bags, respectively.

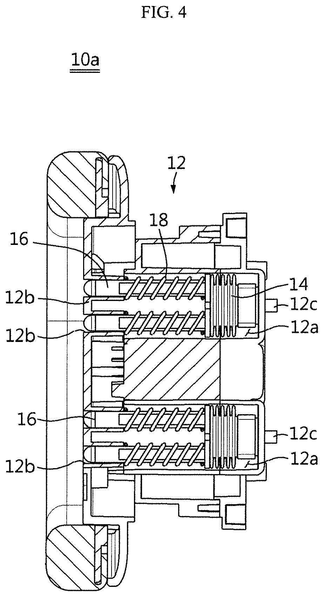

FIG. 4 is a vertical sectioned view, taken along the line A-A' of FIG. 2.

As shown in FIG. 4, the massage module 10a includes a housing 12, a plurality of fluid filling bags 14, and a plurality of stimulation pins 16.

The housing 12 has the facing portion facing the external ear of the user as described above and has the plurality of accommodation holes 12a separated by a barrier therein. The number of accommodation holes 12a in the housing 12 may correspond to the number of unit areas formed by dividing the external ear area according to the stimulation effects or positions. The plurality of fluid filling bags 14 are respectively dispersed and accommodated in the plurality of accommodation holes 12a formed in the housing 12. In addition, the plurality of stimulation pins 16 are disposed inside the housing 12 such that at least one stimulation pin 16 corresponds to each of the plurality of fluid filling bags 14. The facing portion of the housing 12 has a plurality of guide holes 12b formed in one-to-one relationship with the plurality of stimulation pins 16 disposed inside the housing 12.

Each stimulation pin 16 is pushed by the corresponding fluid filling bag 14 and drawn out of the housing 12 by a predetermined length as the length of the corresponding fluid filling bag 14 is increased. Here, the stimulation pin 16 may be guided by the corresponding guide hole 12b and drawn out in a predetermined direction.

In this case, the plurality of stimulation pins 16 are grouped into a plurality of stimulation pin groups so as to correspond to the plurality of unit areas formed by dividing the external ear area according to the stimulation effects or positions, and the plurality of fluid filling bags 14 may be independently extended by a fluid supplied from the outside to draw the plurality of stimulation pins for each stimulation pin group.

To this end, each fluid filling bag 14 may be formed in a bellows structure having a length increased or decreased in a predetermined direction according to the pressure of the fluid filled therein. In addition, the housing 12 may have a plurality of fluid delivery passages 12c for independently delivering fluid supplied from the outside to each fluid filling bag 14. Each fluid delivery passage 12c may deliver the fluid supplied from an external pump to the fluid filling bag 14 accommodated in the accommodation hole 12a of the housing 12 and discharge the fluid filled in the fluid filling bag 14 to the outside of the housing 12.

In this case, each fluid filling bag 14 may include an opening connected to the fluid delivery passage 12c of the housing 12, a bellows structure having a length increased or decreased in a predetermined direction according to the pressure of the fluid delivered through the opening, and a support provided to a terminal of the bellows structure to support the corresponding stimulation pin 16.

In addition, each stimulation pin 16 may include a head supported in contact with the corresponding fluid filling bag 14, and a rod having one end connected to the head and extending in one direction so that the other end thereof is located within the corresponding guide hole 12b. According to an embodiment, at least one of the plurality of stimulation pins 16 disposed inside the housing 12 may further include an auricular meridian point stimulating cap detachably coupled to the other end of the rod. The auricular meridian point stimulating cap may be made of a soft material and may include an auricular meridian point stimulating substance such as germanium or jade.

Meanwhile, the massage module 10a may further include an elastic body 18. If the length of the corresponding fluid filling bag 14, which pushes the stimulation pin 16 drawn out of the housing 12 through the guide hole 12b, is reduced, the elastic body 18 inserts the drawn stimulation pin 16 into the housing by an elastic force thereof. For example, the elastic body 18 may be configured as a coil spring having one end fixed inside the housing 12 and the other end coupled to the stimulation pin 16. In this case, the elastic body 18 may be inserted into the stimulation pin 16 so that one end thereof is supported by the periphery of the guide hole 12b and the other end thereof supports the head of the stimulation pin 16.

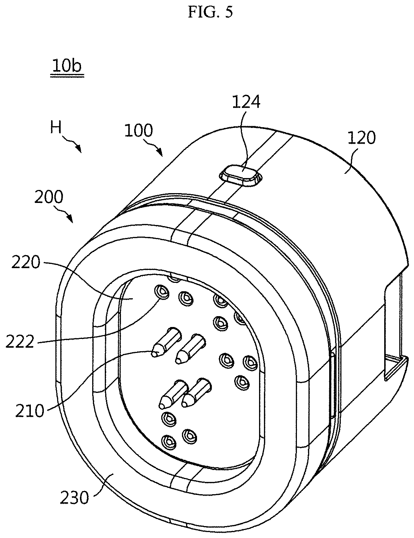

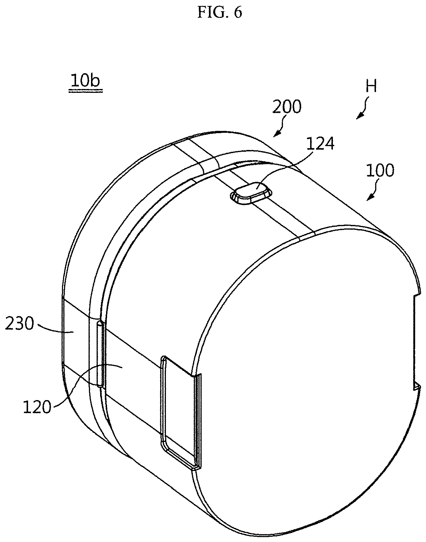

FIGS. 5, 6 and 7 are a front perspective view, a rear perspective view and a front view respectively showing a massage module 10b of an ear therapy device according to another embodiment of the present disclosure.

As shown in FIGS. 5, 6 and 7, the massage module 10b of the ear therapy device according to another embodiment of the present disclosure includes a housing H and a stimulation pin 210.

The housing H has a facing portion facing the external ear of the user, and the facing portion has a guide hole 222 through which the stimulation pin 210 accommodated inside the housing H is drawn out. A plurality of stimulation pins 210 may be accommodated in the housing H and disposed at predetermined positions. In this case, a plurality of guide holes 222 may be formed in the facing portion of the housing H in one-to-one relationship with the plurality of stimulation pins 210.

The massage module 10b is located near the external ear of the user and extracts the stimulation pins accommodated therein to stimulate the external ear of the user. The massage module 10b may include an operating module 100 and an auricular meridian point stimulating module 200.

The operating module 100 drives the auricular meridian point stimulating module 200 and may have a plurality of fluid filling bags whose lengths are independently increased or decreased depending on the pressure of fluid such as air or oil filled in each inner space. As explained later, the operating module 100 may include a plurality of fluid filling bags and a first housing for accommodating the plurality of fluid filling bags.

The auricular meridian point stimulating module 200 accommodates the plurality of stimulation pins 210 having a pin or nail shape for stimulating auricular meridian points of the user and may be detachably coupled to the operating module 100. As explained later, at the massage module 10b of FIG. 5, the user may detach the auricular meridian point stimulating module 200 from the operating module 100 by pressing a separation button 124.

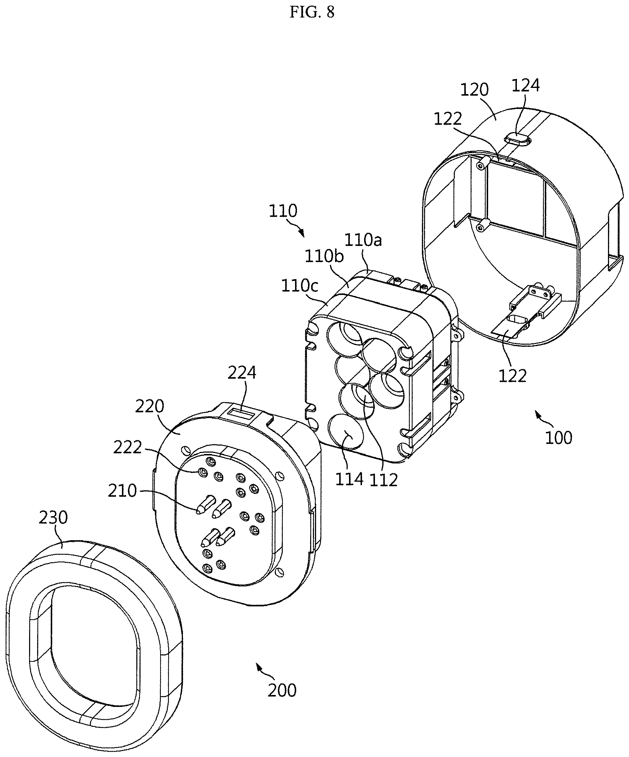

FIG. 8 is an exploded perspective view showing the massage module 10b depicted in FIG. 5.

As shown in FIG. 8, the operating module 100 may include a plurality of fluid filling bags 112 and a first housing 110 accommodating the plurality of fluid filling bags 112.

The first housing 110 has a plurality of accommodation holes 114 to accommodate the plurality of fluid filling bags 112 in the plurality of accommodation holes 114. In this case, the first housing 110 may have a plurality of accommodation holes 114 for independently accommodating the plurality of fluid filling bags 112 and a fluid delivery passage connected to the opening of the fluid filling bag 112 accommodated in the accommodation hole 114 to deliver a fluid to the fluid filling bag 112.

In addition, the first housing 110 may include a plurality of sub housings 110b, 110c stacked and coupled in order. In this case, the plurality of sub housings 110b, 110c may accommodate the plurality of fluid filling bags 112 by dividing the plurality of fluid filling bags 112 in a certain number. For example, the first housing 110 may include a first sub housing 110b having at least one first accommodation hole formed therein to accommodate the fluid filling bag 112 is formed, a penetration hole connected to the first accommodation hole, and a second sub housing 110c having at least one second accommodation hole formed therein to accommodate the fluid filling bag 112 and stacked on the first sub housing 110b.

According to an embodiment, the first housing 110 may further include an external housing 120. The external housing 120 accommodates the sub housings and may have a coupling structure 122 that is detachably coupled to the second housing 220 of the auricular meridian point stimulating module 200.

The second housing 220 of the auricular meridian point stimulating module 200 has a facing portion having a plurality of guide holes 114 formed therein and may be detachably coupled with the first housing 110 to form an inner space in which the plurality of stimulation pins 210 are accommodated.

In this case, the external housing 120 of the first housing 110 may have a coupling structure 122 that is coupled with the second housing 220 of the auricular meridian point stimulating module 200 in a snap-fitting manner. For example, the external housing 120 may have a locking protrusion structure as the coupling structure, and the second housing 220 of the auricular meridian point stimulating module 200 may have a locking groove structure as the corresponding coupling structure 224.

In an embodiment, the second housing 220 of the auricular meridian point stimulating module 200 may be configured to be coupled with the first sub housing 110b or the second sub housing 110c, instead of the external housing 120 of the first housing 110. In addition, the auricular meridian point stimulating module 200 may be coupled with the operating module 100 in various ways allowing each attachment and detachment, such as a simple fitting method or a magnet attachment method.

Since the auricular meridian point stimulating module 200 of the massage module 10b is detachably configured as described above, the auricular meridian point stimulating module may be easily exchanged and customized for each user, and the ear therapy device may be hygienically used and managed even when being commonly used in public facilities such as medical institutions.

The auricular meridian point stimulating module 200 may draw the stimulation pin 210 toward the external ear of the user by means of the fluid filling bag whose length is increased, among the fluid filling bags accommodated in the operating module 100. In this case, the auricular meridian point stimulating module 200 may include a plurality of stimulation pins 210, a second housing 220, and an elastic body explained later. According to an embodiment, the second housing 220 may further include an annular buffering member 230 attached along the edge of the facing portion. In this case, the buffering member 230 may be made of a porous polymer synthetic resin capable of buffering and sound-absorbing, like a sponge.

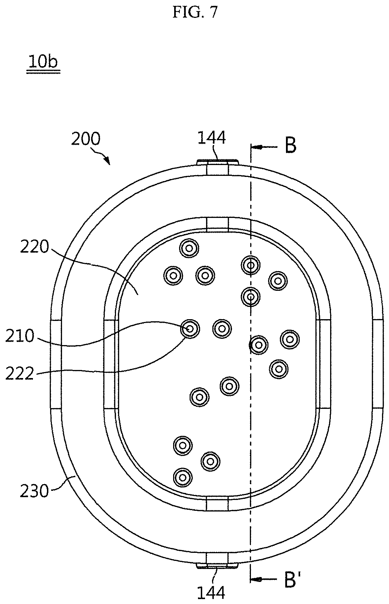

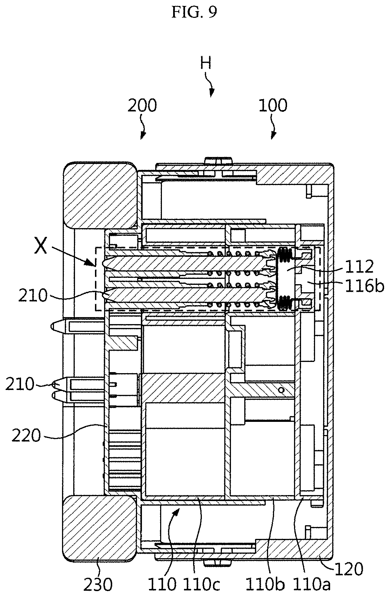

FIG. 9 is a vertical sectioned view, taken along the line B-B' of FIG. 7.

As shown in FIG. 9, the massage module 10a includes a housing H, a plurality of fluid filling bags 112, and a plurality of stimulation pins 210.

The housing H has a facing portion facing the external ear of the user as described above, and has a plurality of accommodation holes divided therein by a barrier. The number of accommodation holes in the housing H may correspond to the number of unit areas formed by dividing the external ear area according to the stimulation effects or positions. The plurality of fluid filling bags 112 are dispersed and accommodated in the plurality of accommodation holes formed in the housing H, respectively. In addition, the plurality of stimulation pins 210 are disposed inside the housing H so that at least one stimulation pin 210 corresponds to each of the plurality of fluid filling bags 112. The facing portion of the housing H has a plurality of guide holes disposed inside the housing in one-to-one relationship with the plurality of stimulation pins 210 H.

Each stimulation pin 210 is pushed by the corresponding fluid filling bag 112 and drawn out of the housing H by a predetermined length as the length of the corresponding fluid filling bag 112 is increased. Here, the stimulation pin 210 may be guided by the corresponding guide hole to be drawn in a predetermined direction.

In this case, the plurality of stimulation pins 210 are grouped into a plurality of stimulation pin groups so as to correspond to the plurality of unit areas formed by dividing the external ear area according to the stimulation effects or positions, respectively, and the plurality of fluid filling bags 112 may be independently extended by a fluid supplied from the outside to draw the plurality of stimulation pins for each stimulation pin group.

To this end, each fluid filling bag 112 may be formed in a bellows structure having a length increased or decreased in a predetermined direction according to the pressure of the fluid filled therein. In addition, the housing H may have a plurality of fluid delivery passages 116b for independently delivering a fluid supplied from the outside to each fluid filling bag 112. Each fluid delivery passage 116b may deliver the fluid supplied from an external pump to the fluid filling bag 112 accommodated in the accommodation hole of the housing H and discharge the fluid filled in the fluid filling bag 112 to the outside of the housing H.

In this case, each fluid filling bag 112 may include an opening connected to the fluid delivery passage 116b of the housing H, a bellows structure having a length increased or decreased in a predetermined direction according to the pressure of the fluid delivered through the opening, and a support provided to a terminal of the bellows structure to support the corresponding stimulation pin 210.

Meanwhile, the massage module 10b may include an operating module 100 and an auricular meridian point stimulating module 200. The first housing 110 of the operating module 100 has a stacked structure in which a plurality of sub housings 110b, 110c are stacked and coupled. According to an embodiment, the first housing 110 may include an external housing 120 accommodating the plurality of sub housings 110b, 110c.

The operating module 100 and the auricular meridian point stimulating module 200 of the massage module 10b may be detachably coupled to form the massage module 10b. In this case, the stimulation pin 210 of the auricular meridian point stimulating module 200 is located in an inner space formed by coupling the operating module 100 and the auricular meridian point stimulating module 200. In addition, one end of the stimulation pin 210 may be supported by the fluid filling bag 112 accommodated in the operating module 100, and the other end of the stimulation pin 210 may be accommodated in the guide hole of the second housing 220. If the fluid delivered from the operating module 100 through the fluid delivery passage 116b of the first housing 110 is filled into the fluid filling bag 112, the length of the fluid filling bag 112 is increased, so that the stimulation pin 210 of the auricular meridian point stimulating module 200 is drawn out of the second housing 220.

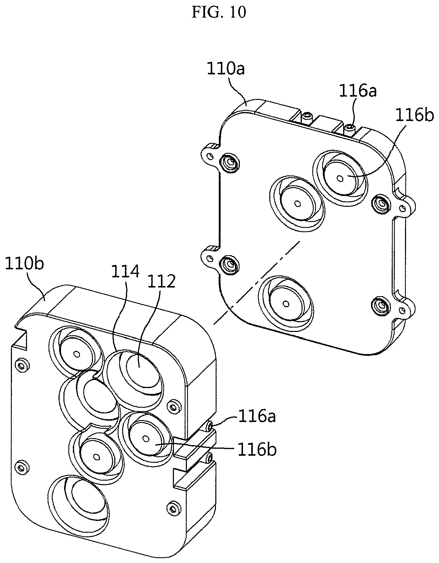

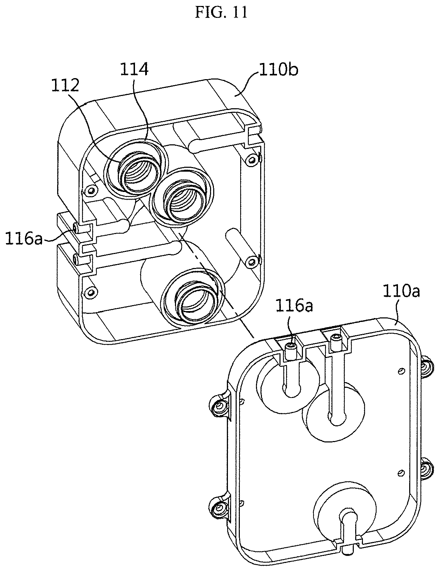

FIGS. 10 and 11 are a front perspective view and a rear front perspective view respectively showing a first layer structure of the first housing 110.

As shown in FIGS. 10 and 11, the first layer structure of the first housing 100 may be formed by coupling a base plate 110a and a first sub housing 110b.

The base plate 110a may have fluid delivery passages 116a, 116b for delivering a fluid to the fluid filling bag 112 accommodated in the first accommodation hole 114 of the first sub housing 110b. That is, the base plate 110a may include an inlet hole 116a for receiving the fluid from the outside and an outlet hole 116b for delivering the fluid introduced through the inlet hole 116a to the fluid filling bag 112. In this case, the inlet hole 116a may be connected with a tubular member (not shown) which forms a moving path of the fluid moved by a pneumatic pump, a hydraulic pump, or the like, and the outlet hole 116b may be connected to the opening of the fluid filling bag 112 accommodated in the first sub housing 110b. As explained later, all of the inlet holes 116a and the outlet holes 116b formed in the first housing 110 are used for independently delivering the fluid to the corresponding fluid filling bags 112, respectively.

The first sub housing 110b may have a first accommodation hole 114 for accommodating the corresponding fluid filling bag 112, and fluid delivery passages 116a, 116b for delivering the fluid to the fluid filling bag 112 accommodated in the second sub housing 110c. The outlet hole 116b of the base plate 110a may be inserted into and coupled to the opening of the fluid filling bag 112 accommodated in the first sub housing 110b.

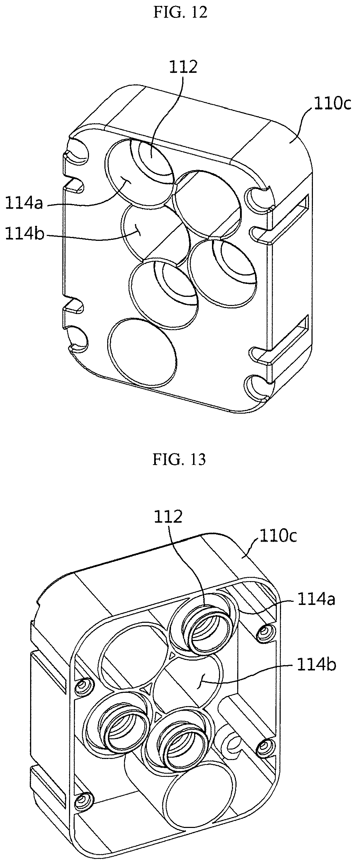

FIGS. 12 and 13 are a front perspective view and a rear front perspective view respectively showing a second layer structure of the first housing.

The second sub housing 110c of the second layer structure of the first housing 110 is stacked on the first sub housing 110b that is stacked on and coupled to the base plate 110a. The second sub housing 110c may have a second accommodation hole 114a for accommodating the corresponding fluid filling bag 112 and a penetration hole 146b through which the fluid filling bag 112 accommodated in the first sub housing 110b passes as its length is increased or decreased according to the pressure of the fluid filled therein. That is, the penetration hole 146b of the second sub housing 110c is formed at a position corresponding to the first accommodation hole 114 of the first sub housing 110b. The outlet hole 116b of the first sub housing 110b may be inserted into and coupled to the opening of the fluid filling bag 112 accommodated in the second sub housing 110c.

Since the first housing 110 accommodates the plurality of fluid filling bags 112 in each layer of the stacked structure in a predetermined number, it is possible to enhance the space utilization of the massage module and reduce the size of the massage module to correspond to the size of the external ear of the user.

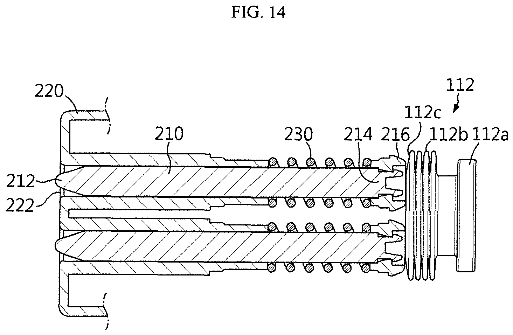

FIG. 14 is a diagram showing a portion X of FIG. 9.

As shown in FIG. 14, the auricular meridian point stimulating module 200 may include a plurality of stimulation pins 210, a second housing 220, and an elastic body 230.

The second housing 220 may have a plurality of guide holes 222 that accommodates at least a part of the stimulation pins 210 and guides the movement of the stimulation pins 210. The guide holes 222 formed in the second housing 220 may be formed in a plurality of groups so as to correspond to the plurality of unit areas formed by dividing the external ear area according to stimulation effects or positions.

The stimulation pin 210 may include a head 216 supported in contact with the corresponding fluid filling bag 112, and a rod 214 having one end connected to the head 216 and extending in one direction so that the other end thereof is located in the guide hole 222. In this case, the head 216 may be formed as a separate member in the form of a cap coupled to the rod 214. The terminal 212 of the rod 214 may be provided in the form of a rounded tip to appropriately stimulate the external ear of the user. The stimulation pin 210 may have a pin or nail shape as a whole.

If the operating module 100 and the auricular meridian point stimulating module 200 are coupled, the rod 214 of the stimulation pin 210 is accommodated in the guide hole 222 and the head 216 of the stimulation pin 210 is supported by the fluid filling bag 112 of the operating module 100.

If the length of the fluid filling bag 112 that pushes the stimulation pin 210 drawn out of the second housing 220 is decreased, the elastic body 230 inserts the drawn stimulation pin 210 into the second housing 220 by an elastic force thereof. To this end, the elastic body 230 may be configured as a coil spring having one end fixed inside the second housing 220 and the other end coupled to the stimulation pin 210. In this case, the elastic body 230 may be inserted into the stimulation pin 210 so that one end thereof is supported by the periphery of the guide hole 222 and the other end thereof supports the head 216 of the stimulation pin 210.

Meanwhile, the fluid filling bag 112 of the operating module 100 may include an opening 112a into which the outlet hole 116b of the first housing 110 is inserted and connected as described above, a bellows structure 112b having a length increased or decreased according to the internal pressure, and a support 112c provided to a terminal of the bellows structure 112b to at least one stimulation pin 210.

Also, a rigid cap may be coupled to the support of the fluid filling bag 14 shown in FIG. 4 or the fluid filling bag 112 shown in FIGS. 9 and 14. In this configuration, it is possible to increase the supporting force of the fluid filling bag 14, 112 and to prevent the fluid filling bag 14, 112 from being damaged. Meanwhile, if the fluid filling bag 14, 112 is configured to directly support the stimulation pin 12, 210 as shown in FIGS. 4, 9 and 14, the elastic force of the fluid filling bag 14, 112 may be exerted to provide a soft feeling to the user of the ear therapy device.

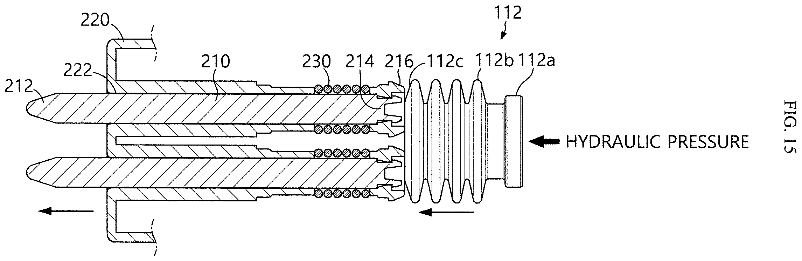

FIG. 15 is a diagram showing a drawing operation of the stimulation pin 210.

As shown in FIG. 15, if the fluid is filled in the fluid filling bag 112 through the opening 112a of the fluid filling bag 112, the internal pressure of the fluid filling bag 112 is increased to extend the length of the bellows structure 112b. As a result, the stimulation pin 210 is pushed in a state of being supported by the support 112c of the fluid filling bag 112 and thus is drawn out of the housing along the guide hole 222. Meanwhile, if the internal pressure of the fluid filling bag 112 is reduced to decrease the length of the fluid filling bag 112, the stimulation pin 210 is inserted into the guide hole 222 again by the elastic force or the restoring force of the elastic body 230. The stimulation pin 210 may be formed of various materials and shapes.

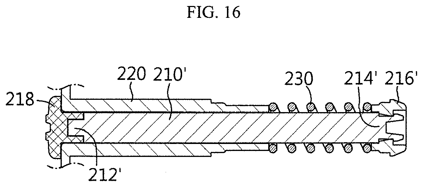

FIG. 16 is a diagram showing a modified example of the stimulation pin.

As shown in 16, one stimulation pin 210' among the plurality of stimulation pins disposed inside the housing H of the massage module 10b may further include an auricular meridian point stimulating cap 218 detachably coupled to a terminal 212' of a rod 214' thereof. The auricular meridian point stimulating cap 218 may be formed of various materials and shapes according to the purpose of use and may be replaced according to the user's choice. For example, the auricular meridian point stimulating cap 218 may be made of a soft polymer synthetic resin and may be configured to include a special substance such as germanium or jade, which may enhance the auricular meridian point stimulation effect.

Meanwhile, in an embodiment, the auricular meridian point stimulating module 200 may include a penetration hole (not shown) formed from the operating module 100 toward the external ear of the user. In this case, the operating module 100 has an external ear stimulating fluid filling bag having a length increasing according to the pressure applied therein to stimulate the external ear of the user through the penetration hole of the auricular meridian point stimulating module 200, like the fluid filling bag 112 described above. If the external ear of the user is stimulated even through the fluid filling bag, it is possible to give both the acupressure effect and the massage effect through the ear therapy device.

In addition, the auricular meridian point stimulating module 200 may have a customized shape in which at least the facing portion facing the external ear of the user is matched with the external ear of the user. According to the present disclosure, the operating module 100 of the massage module and the auricular meridian point stimulating module 200 may be configured to be detachably coupled. Thus, the second housing 220 of the auricular meridian point stimulating module 200 or the like may be separately prepared to be customized for the user through a 3D printer or molding, and the auricular meridian point stimulating module 200 may be coupled to the operating module 100 and used, thereby providing the ear therapy device customized for the user.

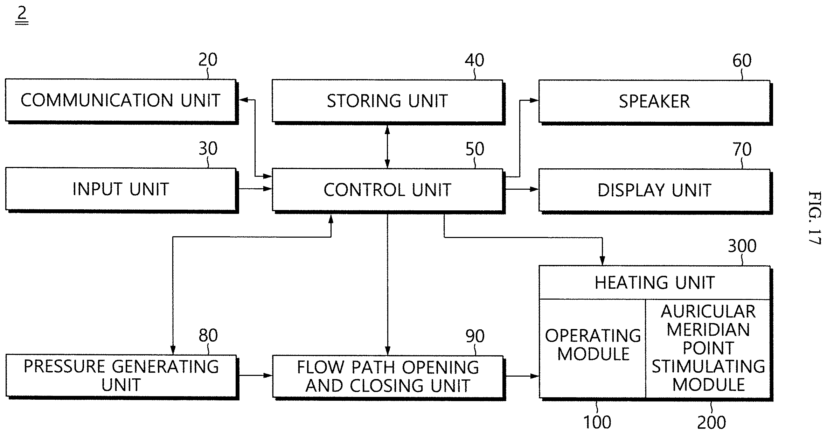

FIG. 17 is a block diagram showing the ear therapy device 2 according to an embodiment of the present disclosure.

As shown in FIG. 17, the ear therapy device 2 may include a pressure generating unit 80, a flow path opening and closing unit 90 and a heating unit 300, together with the massage module 10a, 10b according to any one of the former embodiments, and may further include a communication unit 20 an input unit 30, a storage unit 40, a control unit 50, a speaker 60, a display unit 70 and the like according to an embodiment. In this case, the massage module of the ear therapy device 2 may include the operating module 100 and the auricular meridian point stimulating module 200 described above.

The pressure generating unit 80 may generate a fluid pressure by moving the fluid using a pump. To this end, the pressure generating unit 80 may include a pneumatic pump, a hydraulic pump or the like.

The flow path opening and closing unit 90 may open or close each fluid moving path that connects the pressure generating unit 80 and the massage module 10a, 10b, by using a valve.

The heating unit 300 may transfer heat to the external ear of the user by using a hot wire or a far infrared lamp. According to the present disclosure, since auricular meridian points are stimulated by the stimulation pins 12, 210 while appropriate heat is transferred to the external ear of the user as described above, it is possible to give a thermal massage effect.

Meanwhile, the input unit 30 may include an input means such as a physical operation button or a microphone for voice input, and the input unit 30 may receive a user command for selecting power on/off, an operation mode, stimulation intensity, stimulation time, or the like of the ear therapy device 2 and transmit the user command to the control unit 50.

The communication unit 20 is a wireless communication means using a short range communication technology such as Bluetooth, Near Field Communication (NFC), Infrared Communication (Infrared Communication), or the like. The communication unit 20 may perform the wireless communication with a wireless communication device such as a mobile terminal of the user or a wireless remote controller to receive a user command from the corresponding wireless communication device and transmit the received user command to the control unit 50. In addition, the communication unit 20 may be configured to perform direct communication with a central management server (not shown) or indirectly communication through a mobile communication terminal.

The storing unit 40 may store the fluid filling bag serving as an operation target in each operation mode of the ear therapy device 2 and operation information about the operation contents of the corresponding fluid filling bag. In this case, the storing unit 40 may store an operation information table in which information about the fluid filling bag serving as an operation target according to an operation mode, stimulation intensity, and stimulation time is recorded. To this end, the storing unit 40 may include a fast random access memory and may also include at least one magnetic disk storage device or a nonvolatile memory such as a flash memory device.

The control unit 50 may control the flow path opening and closing unit 90 according to the operation information corresponding to the operation mode selected through the input unit 30, among the operation information for each operation mode stored in the storing unit 40, to selectively open and close each fluid moving path connected to the operating module 100 of the massage module.

The speaker 60 is a sound output means that may output sound of a sound source file reproduced by the control unit 50. The display unit 70 is a visual information display means such as a liquid crystal panel and may display state information of the ear therapy device 2, for example current operation mode information, stimulation intensity, stimulation time, battery state information and the like.

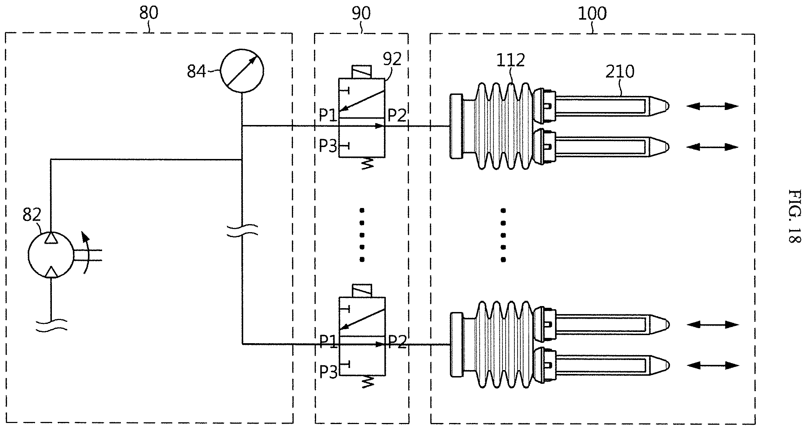

FIG. 18 is a diagram showing a fluid moving path of the ear therapy device 2 according to an embodiment of the present disclosure.

As shown in FIG. 18, the pressure generating unit 80 of the ear therapy device 2 may include a pump 82 for generating a fluid pressure, such as a pneumatic pump or a hydraulic pump, and a pressure sensor 84. The pump 82 may suck in the fluid such as air or oil through the input terminal and discharge the fluid through the output terminal.

The pressure sensor 84 may detect the pressure generated by the pump 82 of the pressure generating unit 80 and transmit the detected pressure value to the control unit 50. In this case, the control unit 50 may control the pressure generating unit 80 according to the pressure value detected by the pressure sensor 84. That is, the control unit 50 may appropriately adjust the pressure transmitted to the fluid filling bag 112 of the operating module 100 by controlling the output of the one-way pump 82 according to the pressure value detected by the pressure sensor 84.

The flow path opening and closing unit 90 of the ear therapy device 2 may include valves 92 in a number corresponding to the fluid filling bags 112 of the operating module 100. In addition, the plurality of valves 92 are respectively installed to the fluid moving paths connecting the pressure generating unit 80 and the plurality of fluid filling bags 112 of the operating module 100 to open and close the corresponding flow path under the control of the control unit 50. In this case, the plurality of valves 92 may be configured as solenoid valves in which a flange is moved by a magnetic force of a coil to open the flow path when electricity is applied and the flange is returned to its original position by gravity or elastic force to block the flow path when the electricity is cut off. Each valve 92 may be configured as a 3-way valve. The 3-way valve is a valve that has three ports and forms two flow paths inside. Each valve 92 may have a first port P1 connected to the output terminal of the pump 82, a second port P2 connected to the fluid filling bag 112 of the operating module 100, and a third port P3 connected to an external side such as a ventilation hole or a fluid storage tank.

For example, if the pressure generating unit 80 generates a pressure and the valve 92 installed to the fluid moving path between the pressure generating unit 80 and the operating module 100 of the massage module is opened, the pressure generated by the pressure generating unit 80 may be transferred to the fluid filling bag 112 of the operating module 100 to increase the length of the fluid filling bag 112, and as a result, the stimulation pin 210 supported by the fluid filling bag 112 may be drawn out.

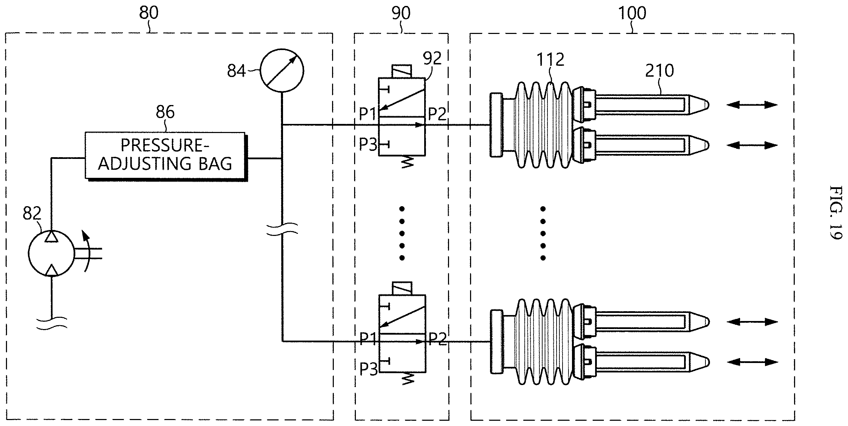

FIG. 19 is a diagram showing a fluid moving path of an ear therapy device 2 according to another embodiment of the present disclosure.

As shown in FIG. 19, the pressure generating unit 80 of the ear therapy device 2 may further include a pressure-adjusting fluid filling bag 86. The pressure-adjusting fluid filling bag 86 is installed to the fluid moving path between the output terminal of the pump 82 and the flow path opening and closing unit 90 to expand or shrink, thereby limiting the pressure change rate generated at the output terminal of the pump 82 to a predetermined threshold value or below when the flow path opening and closing unit 90 performs an opening or closing operation. By installing the pressure-adjusting fluid filling bag 86 to the fluid moving path at the output terminal of the pneumatic motor 82, it is possible to prevent unstable noise of the pump 82 generated by a sudden pressure change.

The operation of the flow path opening and closing unit 90 and the operating module 100 of the ear therapy device 2 may be explained in the same way as the corresponding configuration of FIG. 18.

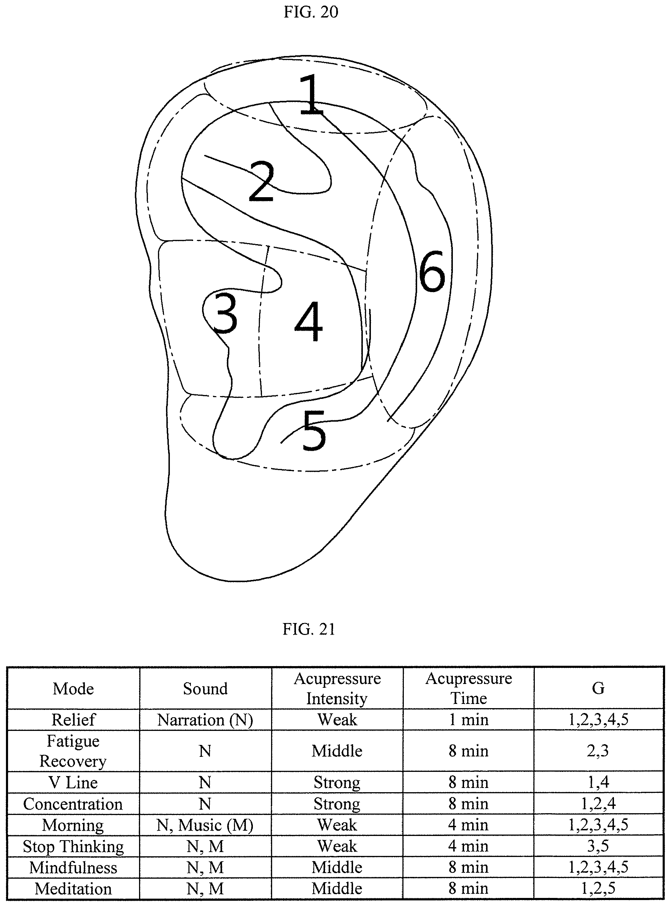

FIG. 20 is a diagram showing an example of stimulation target areas formed by dividing the external ear area of a human according to stimulation effects.

As shown in FIG. 20, the external ear area of the human body has a plurality of meridian points connected to the organs of the body. The meridian points of the external ear area, namely the auricular meridian points, may be divided into six unit areas according to stimulation or massage effects. That is, a first area is an area having auricular meridian points connected to peripheral nerves such as fingers and toes. A second area is an area having auricular meridian points connected to the genitals of the body. A third area and a fourth area are areas having auricular meridian points connect to the internal organs of the body. A fifth area is an area having auricular meridian points connected to the face and brain. A sixth area is an area having auricular meridian points connected to the muscles of the back, arms and shoulders. Since the auricular meridian points are connected to each part and organ of the body as above, the therapeutic effect may be maximized by stimulating auricular meridian points in a specific area with an appropriate intensity for an appropriate time according to the health condition or diagnosis result of the user.

To this end, the plurality of stimulation pins accommodated in the massage module 10a, 10b may be grouped into a plurality of stimulation pin groups respectively corresponding to the unit areas of the external ear as described above, and the plurality of fluid filling bags accommodated in the massage module 10a, 10b may draw stimulation pins in each group, respectively. In this case, the guide holes for guiding the stimulation pins may also be grouped into six groups to respectively correspond to the plurality of unit areas formed by dividing the external ear area of the user.

FIG. 21 is a diagram showing an example of an operation information table stored in the storing unit 40.

As shown in FIG. 21, the storing unit 40 may record and store a fluid filling bag serving as an operation target in each operation mode of the ear therapy device 2 and operation information about operation contents of the corresponding fluid filling bag in a table. In this case, information about a fluid filling bag serving as an operation target according to the operation mode, stimulation intensity and stimulation time may be recorded in the operation information table. In some embodiments, sound information of each operation mode may be further recorded in the operation information table.

In this case, the storing unit 40 may store a sound source file, and the control unit 50 may reproduce a sound source file corresponding to the current operation mode and output the sound through the speaker 60. For example, if the user selects a "fatigue recovery" operation mode among the operation modes of the ear therapy device 2, the control unit 50 may control the ear therapy device 2 with reference to the operation information table of the storing unit 40. That is, the control unit 50 may reproduce a narration (N) sound source file corresponding to the "fatigue recovery" operation mode, and the control unit 50 may control the pressure generating unit 80 and the flow path opening and closing unit 90 to adjust the internal pressure of the fluid filling bags accommodated in the massage module so that only the stimulation pins corresponding to the "fatigue recovery" operation mode are selectively drawn. In this case, the control unit 50 may control the pressure generating unit 80 and the flow path opening and closing unit 90 to synchronize the operation of the ear therapy device 2 with the content or beat of the sound source file that is being reproduced.

In addition, the control unit 50 may collect usage history information such as use location, use time, used sound source and the like of the ear therapy device 2 by the corresponding user, and store the usage history information in the storing unit 40 or transmit to the central management server through the communication unit 20.

As described above, according to the present disclosure, since the stimulation pins accommodated in the massage module are drawn with the appropriate length and pressure by the fluid filling bag to stimulate the external ear of the user, the ear therapy device may massage the external ear of the user in the same way as the actual massage using an acupressure tool, and also the ear therapy device may adaptively operate according to the shape or structure of the external ear that is different for each user.

In addition, instead of simply expanding the fluid filling bags to push the stimulation pins, the stimulation pins are pushed in a predetermined direction using the bellows structure having a length increased or decreased in a predetermined direction according to the pressure of the fluid filled therein, and the guide holes of the housing guide the drawing direction of the stimulation pins pushed by the fluid filling bags. Thus, it is possible to improve the accuracy and uniformity of the stimulation operation using the stimulation pins.

In addition, the plurality of stimulation pins accommodated in the massage module are grouped into a plurality of groups to correspond to the plurality of unit areas formed by dividing the external ear area of the user according to the stimulation effects or positions, and the stimulation pins of each group are drawn according to an operation mode selected by the user to stimulate the external ear of the user. Thus, the user may reflect his/her health status or medical examination results to a massage scenario.

In addition, since the auricular meridian point stimulating module, which accommodates the stimulation pins and faces the external ear of the user, is configured to be detachably coupled with the operating module accommodating the fluid filling bags, the auricular meridian point stimulating module may be easily exchanged and customized for each user, and the ear therapy device may be hygienically used and managed even when being commonly used in public facilities such as medical institutions.

In addition, since the pressure control bag is installed to the output terminal of the pump that delivers the fluid to the fluid filling bag, it is possible to prevent the generation of noise of the pump caused by a rapid pressure change when opening and closing the fluid moving path.

In addition, since the heating unit of the ear therapy device transfers heat to the external ear of the user through a hot wire or a far-infrared lamp, it is possible to give a thermal massage effect through the ear therapy device.

Further, it would be obviously understood that the embodiments according to the present disclosure can also solve various technical objects not mentioned in the specification in the corresponding technical field and also in the related technical fields.

The present disclosure has been explained with reference to specific embodiments. However, it will be clearly understood by those skilled in the art that various modifications can be made within the technical scope of the present disclosure. Therefore, the above-described embodiments should be considered from an illustrative point of view, not from a restrictive point of view. That is, the true scope of the present disclosure is set forth in the appended claims, and all differences within the equivalent scopes thereof should be construed as being included in the present disclosure.

## Claims

1. An ear therapy device, which performs massage to an external ear of a user by using a massage module disposed at a position corresponding to the external ear, wherein the massage module includes:
a housing having a facing portion facing the external ear and a plurality of accommodation holes divided therein by a barrier;
a plurality of fluid filling bags dispersed and accommodated in the plurality of accommodation holes, respectively; and
a plurality of stimulation pins disposed inside the housing such that at least one stimulation pin corresponds to each of the plurality of fluid filling bags,
wherein a plurality of guide holes are formed in the facing portion of the housing in one-to-one relationship with the plurality of stimulation pins,
wherein each fluid filling bag has a bellows structure with a length increased or
decreased in a predetermined direction according to a pressure of a fluid filled therein, and
wherein each stimulation pin is pushed by a corresponding fluid filling bag and drawn out of the housing by a predetermined length when the length of the corresponding fluid filling bag is increased, and the each stimulation pin is guided by a corresponding guide hole to be drawn in a predetermined direction.

2. The ear therapy device according to claim 1,
wherein the housing has a plurality of fluid delivery passages configured to independently deliver a fluid supplied from the outside to the plurality of fluid filling bags, respectively.

3. The ear therapy device according to claim 2,
wherein each fluid filling bag includes:
an opening connected to the fluid delivery passage of the housing;
a bellows structure having a length increased or decreased in a predetermined direction according to the pressure of the fluid delivered through the opening;
and
a support provided to a terminal of the bellows structure to support a corresponding stimulation pin.

4. The ear therapy device according to claim 1,
wherein each stimulation pin includes:
a head supported in contact with the corresponding fluid filling bag; and
a rod having one end connected to the head and extended in one direction so that the other end thereof is located in the corresponding guide hole.

5. The ear therapy device according to claim 4,
wherein at least one of the plurality of stimulation pins further includes an auricular meridian point stimulating cap detachably coupled to the other end of the rod.

6. The ear therapy device according to claim 1,
wherein the plurality of stimulation pins are grouped and disposed into a plurality of stimulation pin groups to respectively correspond to a plurality of unit areas formed by dividing an external ear area according to stimulation effects or positions, and
wherein the lengths of the plurality of fluid filling bags are independently increased by the fluid supplied from the outside to draw the plurality of stimulation pins for each stimulation pin group.

7. The ear therapy device according to claim 1,
wherein the facing portion of the housing includes a protrusion protruding toward the external ear so that a part of the plurality of guide holes is located therein, and
wherein among the plurality of stimulation pins, a stimulation pin drawn through the guide hole located in the protrusion has a greater length than another stimulation pin drawn through a guide hole not in the protrusion.

8. The ear therapy device according to claim 1,
wherein the massage module further includes an elastic body configured to insert the drawn stimulation pin into the housing by an elastic force thereof, when the length of the corresponding fluid filling bag pushing the stimulation pin drawn out of the housing is decreased.

9. The ear therapy device according to claim 1,
wherein the housing includes:
a first housing in which the plurality of accommodation holes are formed to accommodate the plurality of fluid filling bags; and
a second housing having the facing portion in which the plurality of guide holes are formed, the second housing being detachably coupled to the first housing to form an inner space in which the plurality of stimulation pins are accommodated.

10. The ear therapy device according to claim 9,
wherein the first housing includes a plurality of sub housings stacked and coupled in order,
wherein the plurality of sub housings includes:
a first sub housing in which at least one first accommodation hole is formed to accommodate the fluid filling bag; and
a second sub housing in which a penetration hole connected to the first accommodation hole and at least one second accommodation hole accommodating the fluid filling bag are formed, the second sub housing being stacked on the first sub housing.

11. The ear therapy device according to claim 1, further comprising:
a wearable structure coupled to the massage module and put on the head of the user so that the massage module is disposed at a position corresponding to the external ear of the user; and
a heating unit configured to transfer heat to the external ear using a hot wire or a far-infrared lamp.

12. The ear therapy device according to claim 1, further comprising:
an input unit configured to receive a user command to select an operation mode of the ear therapy device;
a pressure generating unit configured to move a fluid by using a pump;
a flow path opening and closing unit configured to open or close each fluid moving path connecting the pressure generating unit and the massage module by using a valve;
a storing unit configured to store a fluid filling bag serving as an operation target according to each operation mode of the ear therapy device and operation information about operation contents of the corresponding fluid filling bag; and
a control unit configured to control the flow path opening and closing unit according to the operation information corresponding to the operation mode selected through the input unit among the operation information of each operation mode stored in the storing unit, so that the fluid moving paths respectively connected to the plurality of fluid filling bags are selectively opened or closed.

13. The ear therapy device according to claim 12,
wherein the pressure generating unit includes:
a pump configured to suck in a fluid through an input terminal and discharge the fluid through an output terminal; and
a pressure control bag installed to a fluid moving path between the output terminal of the pump and the flow path opening and closing unit and expanded or shrunk to limit a pressure change rate, generated at the output terminal of the pump when the flow path opening and closing unit performs the opening or closing operation, to a predetermined threshold value or below.

14. The ear therapy device according to claim 12,
wherein the storing unit stores an operation information table in which information about a fluid filling bag serving as an operation target according to each operation mode, stimulation intensity and stimulation time is stored.
